(19) Europäisches Patentamt
European Patent Office
Office européen des brevets

(11) Numéro de publication: **0 177 392**
**A1**

(12) **DEMANDE DE BREVET EUROPEEN**

(21) Numéro de dépôt: 85401765.4

(22) Date de dépôt: 11.09.85

(51) Int. Cl.[4]: C 07 D 213/72
C 07 D 413/12, C 07 D 407/12
A 61 K 31/495

(30) Priorité: 14.09.84 FR 8414089

(43) Date de publication de la demande:
09.04.86 Bulletin 86/15

(84) Etats contractants désignés:
AT BE CH DE GB IT LI LU NL SE

(71) Demandeur: INNOTHERA
10, avenue Paul Vaillant Couturier
F-94110 Arcueil(FR)

(72) Inventeur: Robba, Max Fernand
4, rue Massillon
F-75004 Paris(FR)

(72) Inventeur: Aurousseau, Michel
104, boulevard Arago
F-75013 Paris(FR)

(74) Mandataire: Kedinger, Jean-Paul et al,
c/o Cabinet Malemont 42, avenue du Président Wilson
F-75116 Paris(FR)

(54) **Nouvelles (pyridyl-2)-1 pipérazines, leur procédé de préparation et leur application en thérapeutique.**

(57) Composés de formule :

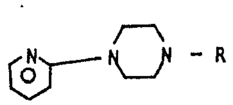
(I)

dans laquelle R représente :

- un groupement cycloalkylméthyle ;
- un groupement 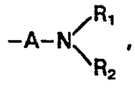 ,

où −A− symbolise une chaîne hydrocarbonée et $R_1$ et $R_2$ désignent chacun un groupe alkyle ou forment conjointement avec N un radical hétérocyclique ;

- un groupement anilinocarbonylméthyle dont le noyau phényle est éventuellement substitué ; ou
- un groupement benzyle substitué.

Ces composés sont utiles comme médicaments à activité cardiotrope.

EP 0 177 392 A1

Croydon Printing Company Ltd.

0177392

Nouvelles (pyridyl-2)-1 pipérazines, leur procédé de préparation et leur application en thérapeutique

La présente invention a pour objet de nouvelles (pyridyl-2)-1 pipérazines substituées en position 4 et leurs sels, ainsi que le procédé de préparation et l'application en thérapeutique de ces composés.

Les nouvelles pipérazines selon l'invention répondent plus précisément à la formule générale :

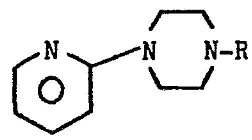

(I)

dans laquelle R représente :

- un groupement cycloalkylméthyle possédant de 4 à 7 atomes de carbone ;
- un groupement de structure $-A-N\genfrac{}{}{0pt}{}{R_1}{R_2}$ où le couple [A, $NR_1R_2$] = [$CH_2CH_2$, $N(iPr)_2$], [$CH_2$ $CH_2$, perhydroazépino], [$CH_2CH_2CH_2$, $N(C_2H_5)_2$], [$CH_2CH_2CH_2$, pyrrolidino], [$CH_2CH_2CH_2$, perhydroazépino], [$CH_2CH_2CH_2$, morpholino], [$CH_2CH_2CH_2$, diméthyl-2,6 morpholino] ou [$CH(CH_3)CH_2$, $N(C_2H_5)_2$] ;
- un groupement anilinocarbonylméthyle dont le noyau phényle est éventuellement substitué par un ou plusieurs atome(s) de fluor ou par un ou plusieurs groupe(s) trifluorométhyle ; ou
- un groupement benzyle substitué par l'enchaînement méthylènedioxy ou par un ou plusieurs groupe(s) méthoxy et éventuellement également par un atome d'halogène.

La présente invention a en outre pour objet, les sels d'addition des composés (I) avec les acides minéraux tels que l'acide chlorhydrique, l'acide sulfurique et l'acide phosphorique ou avec les acides organiques tels que l'acide oxalique, l'acide maléique, l'acide citrique et l'acide méthanesulfonique.

Entre également dans le cadre de la présente invention le procédé de préparation des composés (I) et de leurs sels, procédé qui consiste à condenser la (pyridyl-2)-1 pipérazine de formule :

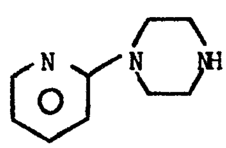

(II)

avec un dérivé de formule :

R - X (III)

où R a la même signification que dans la formule (I) et X représente un groupe électrophile bon partant, notamment un atome de brome ou de chlore ou un groupe mésyloxy ou tosyloxy.

Cette condensation est de préférence réalisée à chaud dans un solvant organique inerte tel que l'alcool isopropylique, l'acétate d'éthyle ou le diméthylformamide. On peut par ailleurs opérer en présence d'un agent de neutralisation des acides tel que le carbonate de sodium ou de potassium par exemple.

Quant aux sels des composés (I), ils sont obtenus de manière tout à fait classique par réaction avec un acide organique ou minéral, de préférence au reflux dans un solvant inerte approprié tel que l'acétone ou le méthanol par exemple.

La préparation ci-après est donnée à titre d'exemple pour illustrer l'invention.

Exemple

(Pyridyl-2)-1 cyclopropylméthyl-4 pipérazine (1)

Une solution de 25 g (0,153 mole) de (pyridyl-2)-1 pipérazine dans 45 ml de diméthyl formamide est additionnée de 16,2 g (0,153 mole) de carbonate de sodium. La suspension est agitée fortement et chauffée à 60° C. On ajoute alors goutte à goutte 20,7 g (0,225 mole) de chlorométhylcyclopropyle, puis on chauffe pendant 4 heures à 80° C avec agitation. Après refroidissement, 200 ml d'eau sont ajoutés puis la phase aqueuse est extraite trois fois avec 100 ml d'éther éthylique. Les phases éthérées réunies sont lavées, séchées sur sulfate disodique anhydre puis concentrées à sec sous vide. On obtient ainsi 30 g (rendement = 90 %) d'une huile jaune constituée par le composé attendu.

Monooxalate (1a)

Le monooxalate est obtenu par réaction de la (pyridyl-2)-1 cyclopropylméthyl-4 pipérazine base avec un équivalent d'acice oxalique par chauffage au

reflux pendant 1 heure dans l'acétone. Ce sel se présente après cristallisation dans l'acétonitrile, sous la forme de cristaux blancs fondant à 198° C.

Bis chlorhydrate (1b)

Le bis chlorhydrate est obtenu par réaction de la (pyridyl-2)-1 cyclopropylméthyl-4 pipérazine base avec une solution d'acide chlorhydrique dans le méthanol. Ce sel présente, après cristallisation dans l'alcool isopropylique, un point de fusion de 265° C.

Les composés répertoriés dans le tableau I ci-après sont préparés par mise en oeuvre de conditions opératoires analogues à celles mises en oeuvre dans l'exemple ci-dessus.

TABLEAU I

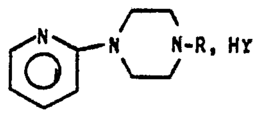

| Numéro de composé | R | HY | Conditions de réaction | point de fusion (°C) | Solvant de cristallisation | Rendement (%) | Formule brute |
|---|---|---|---|---|---|---|---|
| 2 | 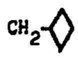 | 1,5 $(COOH)_2$ | DMF, reflux 17h RX=RBr | 179 | Acétonitrile (1) : méthanol (1) | 95 | $C_{17}H_{24}N_3O_6$ |
| 3 | 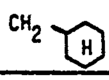 | 2 $(COOH)_2$ | DMF, reflux 17h RX = RBr | 178 | Acétonitrile | 90 | $C_{20}H_{29}N_3O_8$ |
| 4 | 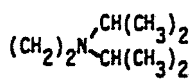 | 3 $(COOH)_2$ | Reflux, 17h isopropanol RX = RCl | 157 | Ethanol à 95° | 95 | $C_{23}H_{36}N_4O_{12}$ |
| | | | | | | | |
| 6 | 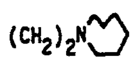 | 3 $(COOH)_2$ | Reflux, 17h isopropanol RX = RCl | 200 | Ethanol à 80° | 90 | $C_{23}H_{34}N_4O_{12}$ |
| 7 | 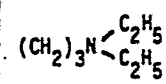 | 3 $(COOH)_2$ | Reflux, 17h isopropanol RX = RCl | 195 | Ethanol à 70° | 90 | $C_{22}H_{34}N_4O_{12}$ |
| 8 | 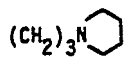 | 3 $(COOH)_2$ | Reflux, 17h isopropanol RX = RCl | 188 | Ethanol à 50° | 95 | $C_{22}H_{32}N_4O_{12}$ |
| | | | | | | | |
| 10 | 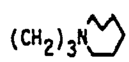 | $(COOH)_2$ | Reflux, 17h isopropanol RX = RCl | 187 | Ethanol à 95° | 90 | $C_{20}H_{32}N_4O_4$ |

TABLEAU I (suite)

| Numéro de composé | R | HY | conditions de réaction | Point de fusion (°C) | Solvant de cristallisation | Rendement (%) | Formule brute |
|---|---|---|---|---|---|---|---|
| 11 | 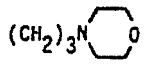 | 2 $(COOH)_2$ | Reflux, 17h isopropanol RX = RCl | 196 | Ethanol à 95° | 70 | $C_{20}H_{30}N_4O_9$ |
| 12 | 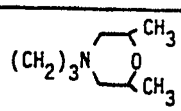 | 2 $(COOH)_2$ | Reflux, 17h isopropanol RX = RCl | 201 | Ethanol à 50° | 95 | $C_{22}H_{34}N_4O_9$ |
| 13 | 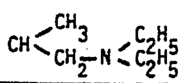 | 3 $(COOH)_2$ | Reflux, 17h isopropanol RX = RCl | 163 | Ethanol à 95° | 90 | $C_{22}H_{34}N_4O_{12}$ |
| 14 | 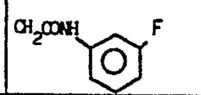 | 1 $(COOH)_2$ | Reflux, 4h DMF | 158 | Ethanol (1); Acétonitrile (1) | 70 | $C_{19}H_{21}FN_4O_5$ |
| 15 | 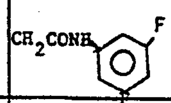 | 1 $(COOH)_2$ | Reflux, 4h DMF RX = RCl | 199 | Méthanol (1); Acétonitrile (1) | 70 | $C_{19}H_{20}F_2N_4O_5$ |
| 16 | 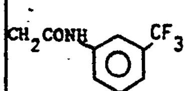 | 2 $(COOH)_2$ | Reflux, 4h DMF RX = RCl | 183 | Méthanol (1); Acétonitrile (1) | 70 | $C_{22}H_{23}F_3N_4O_5$ |
| 17 | 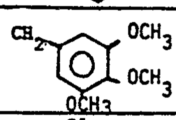 | 2 $(COOH)_2$ | Reflux, 4h DMF RX = RCl | 191 | Acétonitrile | 95 | $C_{23}H_{29}N_3O_{11}$ |
| 18 | 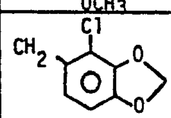 | 1 $(COOH)_2$ | Reflux, 3h DMF RX = RCl | 213 | Acétonitrile | 95 | $C_{19}H_{20}ClN_3O_6$ |

Les composés (I) selon l'invention et leurs sels pharmaceutiquement acceptables ont été testés sur l'animal de laboratoire et se sont montrés doués d'activités pharmacologiques et notamment d'une activité cardiotrope en particulier inotrope positive et bradycardisante.

A/ Mise en évidence in vitro de l'effet bradycardisant

L'effet bradycardisant a été révélé par l'étude de l'action chronotrope sur l'oreillette droite isolée de Cobaye.

Des Cobayes de sexe indifférent, ont été assommés et saignés, l'oreillette droite a été rapidement prélevée et aussitôt immergée dans une solution de Krebs-Henseleit, thermostatée à la température de 36° C ± 0,5° C et aérée par un mélange de $O_2$ et $CO_2$ (95 % et 5 %). La préparation a été reliée à un myographe isométrique (F.50 Narco) puis à un système d'enregistrement (Psysiograph M-K III - Narco).

Un délai de 60 minutes a été nécessaire pour assurer la stabilité d'action chronotrope.

Les composés (I) selon l'invention et leurs sels pharmaceutiquement acceptables ont été ajoutés au milieu nutritif à la concentration de $1.10^{-4}$ $g.ml^{-1}$ et laissés en contact avec l'oreillette 30 minutes. L'action a été appréciée par le pourcentage de diminution du nombre de battements/minutes (batt./mn.) par rapport au nombre de batt./mn. avant l'administration du composé à tester.

Les résultats ont été répertoriés dans le tableau II suivant.

TABLEAU II

Activité bradycardisante des composés selon l'invention étudiée sur l'oreillette droite isolée de Cobaye

| Numéro du composé testé | Diminution de l'effet chronotrope à la concentration de $1.10^{-4}$ $g.ml^{-1}$ % |
|---|---|
| 1a | 24 |
| 2 | 7 |
| 3 | 13 |
| 4 | 9 |
| 6 | Arythmie |
| 7 | 16 |
| 8 | 20 |
| 10 | 0 |
| 11 | Arythmie |
| 12 | 0 |
| 13 | Arythmie |
| 14 | 18 |
| 15 | 0 |
| 16 | 18 |
| 17 | 31 |
| 18 | 11 |

B/ Mise en évidence in vitro et in vivo de l'effet inotrope positif

1/ Etude in vitro

L'étude in vitro a été faite sur la force contractile de l'oreillette gauche isolée de Cobaye. La technique utilisée était celle de P. LUMBEY et Coll. (Cardiovascular Research, II, 17-25, 1977). Elle a permis l'étude de la fonction inotrope $\beta_1$ cardiaque.

Des cobayes de sexe indifférent sont assommés et saignés , l'oreillette gauche étant rapidement prélevée et aussitôt immergée dans une solution de Krebs-Henseleit, thermostatée à la température de 36° C et aérée par un mélange de $O_2$ et de $CO_2$ (95 % et 5 %). La préparation a été reliée à un myographe isométrique (F-50 Narco), puis à un système d'enregistrement (Physiograph M-K III Narco). Pendant toute la durée de l'essai, l'oreillette a été entraînée électriquement par un neurostimulateur (Equipement Industriel II) à la fréquence de 90 à 120 batt./mn.

Les agonistes, c'est-à-dire le produit de référence et les composés selon l'invention,ont été ajoutés aumilieu nutritif suivant la méthode cumulative de VAN ROSSUM (Arch. Inter. Pharmacodyn., 143, 299-330, 1963).

La quantification de l'effet inotrope positif effectuée par le calcul du $pD_2$ [d'après E.J. ARIENS et J.M. VAN ROSSUM - Arch. Intern. Pharmacodyn. CX, n° 2, 2, 19 et Arch. Inter. Pharmacodyn. 143, 299-330, 1963] qui caractérise l'affinité du composé testé pour les récepteurs ainsi que l'activité intrinsèque ($\alpha$) qui définit la puissance relative de l'agoniste par rapport à un produit de référence (DOPAMINE), ont été déterminées.

Les résultats obtenus à titre d'exemple avec le composé 1a selon l'invention, ont été réunis dans le tableau III suivant.

TABLEAU III

Activité inotrope du composé 1a sur l'oreillette isolée de Cobaye

| Produit | $pD_2$ (a) | Kb (b) | α (c) |
|---|---|---|---|
| 1a | 4,68 ± 0,42 | $2,09.10^{-5}$M | 0,60 ± 0,30 |
| DOPAMINE (produit de référence) | 5,13 ± 0,18 | $7,41.10^{-6}$M | 1,00 |

(a) $pD_2$ = log. négatif de la concentration molaire de l'agoniste qui produit un effet égal à 50 % de son action maximum.

(b) Kb = concentration molaire de l'agoniste qui produit un effet égal à 50 % de son action maximum.

(c) α = rapport entre l'effet maximum d'un agoniste et l'effet maximum de l'agoniste de référence (α = 1).

2/ Etude in vivo

On a par ailleurs procédé à l'étude de l'effet inotrope positif des composés (I) selon l'invention et de leurs sels pharmaceutiquement acceptables sur la dynamique cardiaque de l'animal anesthésié et sur l'animal éveillé implanté chroniquement.

a. Etude sur le chien anesthésié au NEMBUTAL

Deux protocoles d'étude ont été réalisés, l'un sur le chien à thorax ouvert, l'autre sur le chien à thorax fermé. Les pressions intraventriculaires gauches (PIVG) et aortiques (sus sigmoïdiennes) (PAO) ont été mesurées avec des microcapteurs haute fidélité (Millar 5F), la dérivée première de la Pression Ventriculaire Gauche (dP/dt) a été enregistrée ainsi que l'électrocardiogramme. La $PCO_2$ alvéolaire instantanée a été mesurée en continu. Enfin, divers débits sanguins ont été mesurés, à savoir le débit fémoral et carotidien chez les animaux à thorax fermé, aortique et coronarien chez ceux où une thoracotomie a été pratiquée. Ces divers débits ont été mesurés par la méthode électromagnétique.

RESULTATS

Etude chez l'animal à thorax ouvert

A titre d'exemples, deux doses du composé 1a ont été testées : 0,5 et 1 $mg.kg^{-1}$ i.v. Les résultats sont répertoriés dans le tableau IV ci-après.

Pour la dose de 0,5 $mg.kg^{-1}$ i.v., le débit aortique s'accroît significativement de 21 % et il en est de même du volume d'éjection systolique qui s'accroît de 16 %, l'acmé de l'action se situant 45 minutes après l'injection. Une augmentation non significative de 15 % de la dP/dt est observée. Parallèlement, la pression télédiastolique ventriculaire gauche diminue significativement (28 %).

Les autres paramètres mesurés ne varient pas significativement.

Pour la dose de 1 $mg.kg^{-1}$ i.v., les résultats sont approximativement similaires à ceux observés à la dose inférieure, à l'exception de la dP/dt positive qui s'accroît significativement (+ 31 %) durant les 90 minutes qui suivent l'injection.

Etude chez l'animal à thorax fermé

Une analyse de la relation dose/effet sur trois chiens (voir résultats répertoriés dans le tableau V ci-après) confirme les résultats obtenus chez le chien à thorax ouvert pour le composé 1a.

L'acmé de l'effet inotrope positif du composé 1a apparaît dès la dose de 0,5 $mg.kg^{-1}$ i.v., l'augmentation de la posologie n'accroît pas l'effet. L'activité inotrope positive chez l'animal à thorax fermé est plus intense pour une posologie de 0,5 $mg.kg^{-1}$ que chez l'animal à thorax ouvert ; il y a une augmentation de la dP/dt + maximale de 52 %. La durée d'action de l'effet inotrope positif excède 3 heures.

Il est important de souligner que le composé 1a possède un effet analeptique respiratoire net et durable.

TABLEAU IV

Effet du composé 1a sur l'hémodynamique cardiaque chez le chien anesthésié (nombre d'animaux utilisés pour le test : 3)

Animaux à thorax ouvert

| | Modification 0,5 $mg.kg^{-1}$ i.v. (en %) | Modification 1 $mg.kg^{-1}$ i.v. (en %) |
|---|---|---|
| D.A.O. [a] | + 21 | + 23 |
| V.E.S. [b] | + 16 | + 13 |
| dP/dt+ [c] | + 15 | + 31 |

TABLEAU V

Effet du composé 1a sur l'hémodynamique cardiaque chez le chien anesthésié (nombre d'animaux utilisés pour le test : 3)

Animaux à thorax fermé

| | Modification 0,5 $mg.kg^{-1}$ i.v. (en %) | Modification 2 $mg.kg^{-1}$ i.v. (en %) |
|---|---|---|
| P.Ao m [d] | + 11 | + 8 |
| dP/dt+ [c] | + 52 | + 28 |
| PtdVG [e] | - 35 | - 31 |

[a] D.A.O. : Débit aortique

[b] V.E.S. : Volume d'éjection systolique

[c] dP/dt+ : Dérivée première de la pression ventriculaire gauche

[d] P.Ao m : Pression Aortique moyenne

[e] Pression Télédiastolique Ventriculaire Gauche

b. Etude chez le chien éveillé chroniquement implanté

Les composés ont été testés par voie orale et intraveineuse sur des chiens préalablement implantés, avec une sonde de pression placée dans le ventricule gauche (Janssen) et un capteur de débit Doppler placé sur l'aorte, les essais étant réalisés au minimum 8 jours après l'implantation.

RESULTATS

Voie orale (voir tableau VI)

Le composé 1a a été étudié à deux doses : 5 et 10 $mg.kg^{-1}$ (voie orale). On observe un accroissement des performances cardiaques traduit par l'augmentation de la dP/dt + (57 %), de la dP/dt+ (32 %), du débit aortique (27 %). Parallèlement, la fréquence cardiaque s'accroît de 31 %. P

Les résultats sont approximativement similaires pour les deux doses.

Voie intraveineuse

Le composé 1a a été étudié à deux doses : 0,5 et 1 $mg.kg^{-1}$. L'effet semble, dans ce cas, dépendant de la dose et se caractérise, comme pour la voie orale, par une augmentation des performances de la dynamique cardiaque, l'accroissement de la dP/dt + atteignant 136 % pour la dose de 1 $mg.kg^{-1}$.

TABLEAU VI

Effet du composé 1a sur l'hémodynamique cardiaque chez le chien éveillé chroniquement implanté (nombre d'animaux pour le test : 3)

| Chien non anesthésié chroniquement implanté | Modification 5 $mg.kg^{-1}$ p.o. (en %) | Modification 10 $mg.kg^{-1}$ p.o. (en %) |
|---|---|---|
| dP/dt + [(a)] | + 57 | + 55 |
| D.A.O. [(b)] | + 27 | + 27 |
| PIVG Syst. [(c)] | + 15 | + 26 |
| F.C. [(d)] | + 31 | + 45 |

[(a)] dP/dt+ : Dérivée Première de la Pression Ventriculaire gauche

[(b)] D.A.O. : Débit Aortique

[(c)] PIVG Syst. : Pression Intraventriculaire Gauche systémique

[(d)] F.C. : Fréquence Cardiaque

La toxicité des composés selon l'invention a également été étudiée. Il s'agit plus précisément de l'étude de la toxicité par administration unique qui a été réalisée pour tous les composés, chez la souris (5 animaux par lot) de sexe mâle, E.O.P.S. de souche SWISS.

Les composés à tester ont été solubilisés dans une solution aqueuse à 9°/₀₀ de NaCl, à des concentrations variables et les solutions obtenues ont été injectées par voie veineuse à raison de 10 $ml.kg^{-1}$ de poids corporel.

L'administration a été faite en 20 secondes, les animaux étant gardés en observation clinique pendant une durée de 3 heures après l'amdministration, puis quotidiennement pendant 7 jours.

Les doses létales 50 % ($DL_{50}mg.kg^{-1}$) ont été calculées par la méthode de Litchfield et Wilcoxon (J. Pharm. Exp. Therap., 96-99, 1949) à partir des diverses mortalités constatées.

Les résultats de cette étude sont répertoriés dans le tableau VII.

TABLEAU VII

Toxicité par administration unique chez la Souris par voie intraveineuse

| N° des composés | $DL_{50}$ mg.kg$^{-1}$ (a) 95% limite confiance | Symptomatologie clinique (b) |
|---|---|---|
| 1a | 47 ( 43 - 52 ) | - Sédation puis phénomène de Straub, cris, convulsions. |
| 2 | 67 ( 58 - 78 ) | - Tonneaux, convulsions, salivation. |
| 3 | 81 ( 74 - 89 ) | - Hypersensibilité, stéréotypie. |
| 4 | 95 ( 87 -104 ) | - Cyanose, sursauts. |
| | | |
| 6 | 84 ( 78 - 91 ) | - Convulsions, sauts, phén. de Straub. |
| 7 | 96 ( 81 -113 ) | - Cyanose, apnée |
| 8 | 142 (129 -156 ) | - Polypnée, agitation, stéréotypie |
| | | |
| 10 | 74 ( 67 - 82 ) | - Apnée, convulsions, hypersensibilité. |
| 12 | 149 (136 -163 ) | - Convulsions, phén. de Straub, tremblements, salivation. |
| 13 | 76 ( 64 - 90 ) | - Hypersensibilité, exophtalmie, début de convulsions. |
| 14 | 129 (118 -141 ) | - Apnée, sédation |
| 16 | 109 ( 99 -121 ) | - Apnée, phén. de Straub, convulsions, tonneaux, salivation, lacrymation. |
| 17 | 130 (122 -139 ) | - Apnée, phén. de Straub, convulsions. |

(a) *$DL_{50}$ calculée par la méthode de Litchfield et Wilcoxon.*

(b) *Symptômes cliniques observés à la dose où il n'y a pas de mortalité.*

L'étude pharmacologique ci-dessus montre l'intérêt thérapeutique des composés (I) et de leurs sels pharmaceutiquement acceptables. Ces composés et sels trouvent donc leur application en tant que médicaments pour l'homme et l'animal en particulier pour le traitement des maladies cardiaques et notamment des insuffisances cardiaques (défaillances et troubles caractérisés par un débit cardiaque trop bas).

La présente invention s'étend par ailleurs aux compositions pharmaceutiques contenant au moins l'un des composés (I) et sels d'addition d'acide pharmaceutiquement acceptables de ces composés (I), ainsi qu'un ou plusieurs véhicule(s) pharmaceutiquement acceptable(s). Ces compositions peuvent être formulées notamment en vue de leur administration orale et se présenter alors par exemple sous forme de dragées, gélules, comprimés ou solutions buvables, ou en vue de leur administration parentérale et se présenter alors sous la forme de solutions injectables, les méthodes de préparation de ces différentes formes étant bien connues de l'homme de l'art.

Les médicaments et les compositions pharmaceutiques selon l'invention peuvent être administrées, en une ou plusieurs prises, à des doses quotidiennes correspondant à une quantité de principe(s) actif(s) pouvant aller, selon les cas, de 50 mg à 1 g.

Revendications (pour les états contractants : BE, CH, DE, GB, IT, LI, LU, NL, SE)

1. (Pyridyl-2)-1 pipérazines répondant à la formule générale :

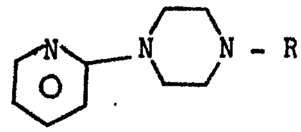

(I)

dans laquelle R représente :

- un groupement cycloalkylméthyle possédant de 4 à 7 atomes de carbone ;
- un groupement de structure $-A-N\genfrac{}{}{0pt}{}{R_1}{R_2}$ où le couple [A, $NR_1R_2$] = [$CH_2CH_2$, $N(iPr)_2$], [$CH_2$ $CH_2$, perhydroazépino], [$CH_2CH_2CH_2$, $N(C_2H_5)_2$], [$CH_2CH_2CH_2$, pyrrolidino], [$CH_2CH_2CH_2$, perhydroazépino], [$CH_2CH_2CH_2$, morpholino], [$CH_2CH_2CH_2$, diméthyl-2,6 morpholino] ou [$CH(CH_3)CH_2$, $N(C_2H_5)_2$] ;
- un groupement anilinocarbonylméthyle dont le noyau phényle est éventuellement substitué par un ou plusieurs atome(s) de fluor ou par un ou plusieurs groupe(s) trifluorométhyle ; ou
- un groupement benzyle substitué par l'enchaînement méthylènedioxy ou par un ou plusieurs groupe(s) méthoxy et éventuellement également par un atome d'halogène;

ainsi que leurs sels d'addition d'acide minéral ou organique.

2. Composés selon la revendication 1, pour lesquels R = cyclopropylméthyle, cyclobutylméthyle ou cyclohexyméthyle.

3. Composés selon la revendication 1, pour lesquels R est soit un groupement anilinocarbonylméthyle dont le noyau phényle porte un substituant fluor en position 3, deux substituants fluor respectivement en positions 3 et 5 ou un substituant trifluorométhyle en position 3, soit un groupement triméthoxy-3,4,5 benzyle ou un groupement chloro-2 méthylènedioxy-3,4 benzyle.

4. Médicament notamment à activité cardiotrope, caractérisé en ce qu'il est constitué par l'une des (pyridyl-2)-1 pipérazines de formule (I) objet de l'une quelconque des revendications précédentes ou par l'un des sels pharmaceutiquement acceptables de ces (pyridyl-2)-1 pipérazines.

5. Composition pharmaceutique, caractérisée en ce qu'elle comprend au moins un élément choisi parmi les (pyridyl-2)-1 pipérazines de formule (I) objet de l'une des revendications 1 à 3, et les sels pharmaceutiquement acceptables de ces (pyridyl-2)-1 pipérazines, en combinaison avec un véhicule pharmaceutiquement acceptable.

6. Procédé de préparation des (pyridyl-2)-1 pipérazines et de leurs sels d'addition d'acide selon la revendication 1, caractérisé en ce qu'il consiste à condenser la (pyridyl-2)-1 pipérazine de formule :

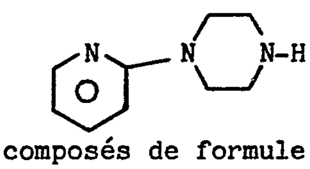

(II)

respectivement avec les composés de formule :

R - X (III)

où R a la même signification que dans la revendication 1 et X est un groupe électrophile bon partant, puis éventuellement à salifier les composés (I) ainsi obtenus.

7. Procédé selon la revendication 6, caractérisé en ce que X = Cl ou Br et en ce que l'on opère en présence d'un agent de neutralisation des acides et d'un solvant organique inerte.

Revendications (Etat contractant : AT)

1. Procéde de préparation de (pyridyl-2)-1 pipérazines répondant à la formule générale :

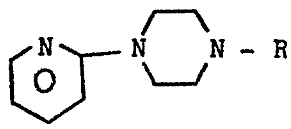

(I)

dans laquelle R représente :

- un groupement cycloalkylméthyle possédant de 4 à 7 atomes de carbone ;
- un groupement de structure $-A-N<^{R_1}_{R_2}$ où le couple [A, $NR_1R_2$] = [$CH_2CH_2$, $N(iPr)_2$], [$CH_2$ $CH_2$, perhydroazépino], [$CH_2CH_2CH_2$, $N(C_2H_5)_2$], [$CH_2CH_2CH_2$, pyrrolidino], [$CH_2CH_2CH_2$, perhydroazépino], [$CH_2CH_2CH_2$, morpholino], [$CH_2CH_2CH_2$, diméthyl-2,6 morpholino] ou [$CH(CH_3)CH_2$, $N(C_2H_5)_2$] ;
- un groupement anilinocarbonylméthyle dont le noyau phényle est éventuellement substitué par un ou plusieurs atome(s) de fluor ou par un ou plusieurs groupe(s) trifluorométhyle ; ou
- un groupement benzyle substitué par l'enchaînement méthylènedioxy ou par un ou plusieurs groupe(s) méthoxy et éventuellement également par un atome d'halogène;

ainsi que de leurs sels d'addition d'acide minéral ou organique, caractérisé en ce qu'il consiste à condenser la (pyridyl-2)-1 pipérazine de formule :

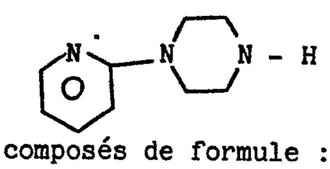

(II)

respectivement avec les composés de formule :

$$R - X \qquad (III)$$

où R a la même signification que dans la formule (I) et X est un groupe électrophile bon partant, puis éventuellement à salifier les composés de formule (I) ainsi obtenus.

2. Procédé selon la revendication 1, caractérisé en ce que X = Cl, Br, mésyloxy ou tosyloxy.

3. Procédé selon la revendication 1 ou 2, caractérisé en ce que l'on opère à chaud dans un solvant organique inerte choisi parmi l'alcool isopropylique, l'acétate d'éthyle et le diméthylformamide.

4. Procédé selon la revendication1, 2 ou 3, caractérisé en ce que l'on opère en présence d'un agent de neutralisation des acides.

5. Composition pharmaceutique, caractérisée en ce qu'elle comprend au moins un élément choisi parmi les (pyridyl-2)-1 pipérazines de formule (I) définie à la revendication 1 et les sels pharmaceutiquement acceptables de ces (pyridyl-2)-1 pipérazines, en mélange avec un véhicule pharmaceutiquement acceptable.

Office européen des brevets

# RAPPORT DE RECHERCHE EUROPEENNE

0177392
Numéro de la demande

EP 85 40 1765

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| X | FR-A-2 533 564 (SELVI & CO. S.p.A.)<br>* Pages 16-21; pages 28-60 *<br>----- | 1,3-10 | C 07 D 213/72<br>C 07 D 413/12<br>C 07 D 407/12<br>A 61 K 31/495 |
| | | | **DOMAINES TECHNIQUES RECHERCHES (Int. Cl.4)** |
| | | | C 07 D 213/00<br>C 07 D 413/00<br>C 07 D 407/00<br>A 61 K 31/00 |

Le présent rapport de recherche a été établi pour toutes les revendications

| Lieu de la recherche | Date d'achèvement de la recherche | Examinateur |
|---|---|---|
| LA HAYE | 06-12-1985 | VERHULST W. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03 82